# EUROPEAN PATENT APPLICATION

(11) **EP 1 970 039 A1**
(43) Date of publication of application: **17.09.2008**
(21) Application number: 06843192.3
(22) Date of filing: 25.12.2006
(51) Int. Cl.: A61J 1/05, A61N 1/30

(54) **DEVICE FOR TRANSDERMAL PREPARATION**

(30) Priority: 27.12.2005 JP 2005374400
(71) Applicant: Hisamitsu Pharmaceutical Co., Inc., Tosu-shi, Saga 841-0017 (JP); Kyodo Printing Co., Ltd., Tokyo-to (JP)
(72) Inventor: TOKUMOTO, Seiji, Tsukuba-shi, Ibaraki 3050856 (JP); ADACHI, Hirotoshi, Tsukuba-shi, Ibaraki 3050856 (JP); FUCHITA, Yasushi, Tokyo 1128501 (JP); TAKAHASHI, Saori, Tokyo 1128501 (JP); OGAWA, Tatsuya, Tokyo 1128501 (JP)
(74) Representative: Shindler, Nigel
(86) International application number: PCT/JP2006/325789
(87) International publication number: WO 2007/077798

(57) **Abstract**

To provide an easily producible mass-production type transdermal drug device having a structure that a dissolution liquid and a drug can be mixed by simple operations. The device comprises an electrode holder (1) having an electrode layer (1b) formed on a substrate (1a), a drug-loaded member (2) provided on a surface of the electrode holder (1) for forming the electrode layer (1b), and a dissolution liquid-storing container (5) provided on a surface of the electrode holder (1) for not forming the electrode layer (1b), wherein a dissolution liquid passing hole (8) is formed in the electrode holder (1), a flange (5a) of the dissolution liquid-storing container (5) is bonded to the electrode holder (1) via a lid material (6) covering the dissolution liquid passing hole (8), the dissolution liquid-storing container (5) has a protrusion (5b) opposing the dissolution liquid passing hole (8), and the protrusion (5b) has a recessed surface at the tip.

## Description

### TECHNICAL FIELD

The present invention relates to a transdermal drug device used in the medical field of treatment or diagnosis.

### BACKGROUND ART

The iontophoresis is a type of method for enhancement of physical absorption of a drug and administers the drug through the skin or the mucosa by applying voltage to the skin or the mucosa and electrically causing migration of the drug.

In a case where a drug which is particularly poor in stability to water is used in the iontophoresis device, it is necessary to separate the drug and a dissolution liquid from each other at the time of storage in order to prevent the drug from being deteriorated during its storage and to mix the drug and the dissolution liquid immediately before the use for treatment. For that, it is convenient to have a structure that a dissolution liquid-storing container is integrated with an iontophoresis electrode itself which includes an electrode layer to be applied to the skin or the mucosa, and the dissolution liquid and the drug can be mixed by a simple operation.

For example, Patent Literature 1 proposes a structure that a capsule 103 in which an electrolyte solution is encapsulated is fitted to a plaster structure for iontophoresis which is composed of an electrode layer 101 and a drug-containing layer 102 via a film such as an aluminum foil.

### [Patent Literature 1]

Japanese Patent Laid-Open Publication No. Sho 63-102768

### SUMMARY OF THE INVENTION

### Problems to be solved by the Invention

According to the structure proposed by Patent Literature 1, the capsule 103 is pushed to break the film such as an aluminum foil or the like by a projection 103a formed on the capsule 103 so to migrate the contained electrolyte toward the drug-containing layer 102, thereby allowing to mix the electrolyte and the drug.

But, the projection shape of Patent Literature 1 has a problem of decreasing an amount of drug absorption because the film is not broken easily, the solution migration rate is low and the solution tends to remain in the container.

The same problem is also true in a transdermal drug device which has the same structure as the above-described iontophoresis device except that the electrode is not used and mixes the dissolution liquid and the drug just before treatment for absorption through the skin or the mucosa.

The present invention has been made to overcome the above problems and has an object to provide a transdermal drug device which has a structure capable of easily mixing a dissolution liquid and a drug just before treatment, has a high dissolution liquid migration rate and can express a desired drug efficacy.

### Means for solving the Problems

The present invention configured to remedy the above-described problems has the following structures.
(1) A transdermal drug device, comprising a holder having a dissolution liquid passing hole, a drug-loaded member provided on one side surface of the holder, and a dissolution liquid-storing container provided on the other side surface of the holder via a lid member for covering the dissolution liquid passing hole, wherein the dissolution liquid-storing container has a projected portion which is opposed to the dissolution liquid passing hole, and the tip of the projected portion is formed on a recessed surface.
(2) The transdermal drug device according to (1), wherein the projected portion has a top and bottom on its outer circumference.
(3) The transdermal drug device according to (2), wherein the top and the bottom are respectively provided in plural and arranged on the outer circumference of the projected portion in contrast to one another.
(4) The transdermal drug device according to any of (1) through (3), wherein the holder is an iontophoresis device which is provided with an electrode on the drug-loaded member side.

### EFFECTS OF THE INVENTION

According to the transdermal drug device of the present invention, the tip of the projected portion of the dissolution liquid-storing container is formed into a recessed shape, so that when the dissolution liquid-storing container is pushed, the initial contact between the projected portion and the lid member can be made not a plane contact but a line contact, and the lid member can be subject to initial rupture effectively by the concentration of stress to it, and the lid member can be broken through along a large area according to the contour of the projected portion. In other words, the lid member can be broken through along a large range without fail, so that the dissolution liquid migration rate can be increased, and a desired drug efficacy can be realized.

In a case where the outer circumference of the projected portion is provided with a top and bottom, the initial contact between the projected portion and the lid member can be changed from a line contact state to approximately a point contact state, and the lid member can be broken through more effectively.

And, in a case where the top and the bottom are respectively provided in plural and arranged opposite to the outer circumference of the projected portion, the lid member can be subject to initial rupture equally at the plural portions, and the lid member can be broken through along a large range more securely.

### BEST MODE FOR CARRYING OUT THE INVENTION

Embodiments of the present invention are described below taking as an example an iontophoresis device to which the present invention is suitably applied with reference to the drawings.

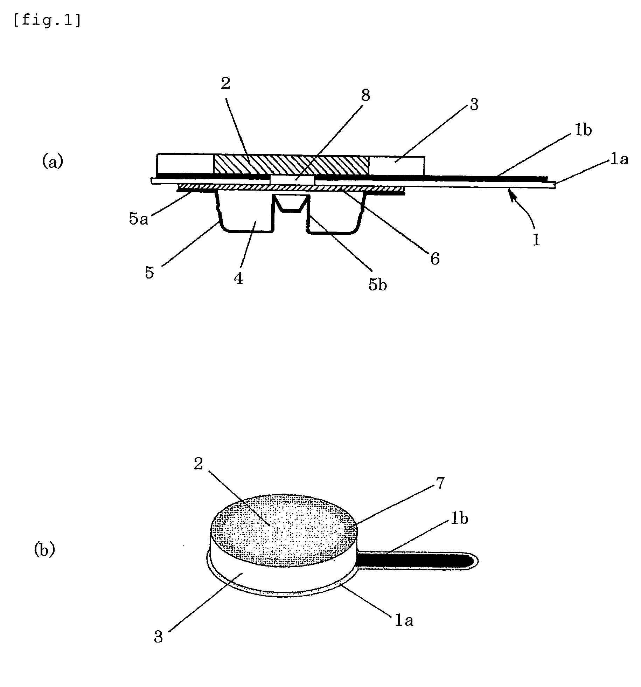

Fig. 1 is a diagram showing an example of the iontophoresis device according to an embodiment of the present invention, where (a) is a sectional view and (b) is a perspective view. This iontophoresis device is mainly comprised of an electrode holder 1 which has an electrode layer 1b formed on a base 1a, a drug-loaded member 2 which is arranged on the electrode holding side of the electrode holder 1, an expanded sheet 3 formed along the circumference of the drug-loaded member 2, a dissolution liquid-storing container (dissolution liquid-storing blister container) 5 in which a dissolution liquid 4 is charged, and a lid member 6 which functions as a lid member of the dissolution liquid-storing container 5. This device is a device for a donor, and a device for reference is separately provided.

### (Electrode holder)

As the base 1a of the electrode holder 1, there are, for example, plastic films of polyethylene terephthalate (PET), polyimide, polyamide, polypropylene and the like, and the polyethylene terephthalate is particularly suitably used because it is excellent in insulation properties, heat resistance, machinability and the like. And, such films may be used as a single film or a composite film.

The electrode layer 1b is composed of a substantially circular part which becomes an electrode discharge portion and an extended part which becomes an electrode terminal portion. As a material for the electrode layer 1b, for example, materials based on metal and nonmetal conductive materials such as silver, silver chloride, carbon, titanium, platinum, gold, aluminum, iron, nickel and a mixture of them can be used. A conductive paste based on such a material may also be used. Such a conductive paste can be used to form the electrode layer by screen printing suitable for mass production.

For the electrode holder 1 having the electrode layer 1b formed on the base 1a, a hole is formed by punching fabrication and it becomes a dissolution liquid passing portion 8 to move the dissolution liquid 4 in the dissolution liquid-storing container 5 to the drug-loaded member 2 at the time of use.

### (Drug-loaded member)

The material for the drug-loaded member 2 is not limited to a particular one if it is a hydrophilic base and can absorb and hold a drug solution, and may be a cellulose fiber, a rayon fiber, a nylon fiber, a polyurethane foam, a polycarbonate foam, a polyvinyl alcohol foam, a polyester foam, a polyester nonwoven fabric, a polyester nonwoven fabric, cotton or a composite of them.

### (Expanded sheet)

The expanded sheet 3 has a function to prevent the drug solution from externally leaking out of the device when the drug-loaded member 2 is impregnated with the dissolution liquid. Therefore, it is required to be securely adhered to the electrode holder 1. And, since the device is used in a state attached to the skin, it is desired that the expanded sheet is a flexible soft foam having as a substrate various types of polymers such as polyurethane, polyethylene, polyvinyl chloride, polychloroprene and acrylic resin foams. And, since it is attached to the skin when used, it is desirable to have an adhesive 7 such as rubber-based, acrylic-based, silicone-based, polyvinyl-based, polyester-based, polyurethane-based or the like coated onto one side.

The drug-loaded member 2 and the expanded sheet 3 can be bonded to the electrode holder 1 with a sealing layer, which is formed of an adhesive material or a heat-sealable material, provided on a prescribed region such as a peripheral portion of the electrode layer 1b of the electrode holder 1.

### (Dissolution liquid-storing blister container)

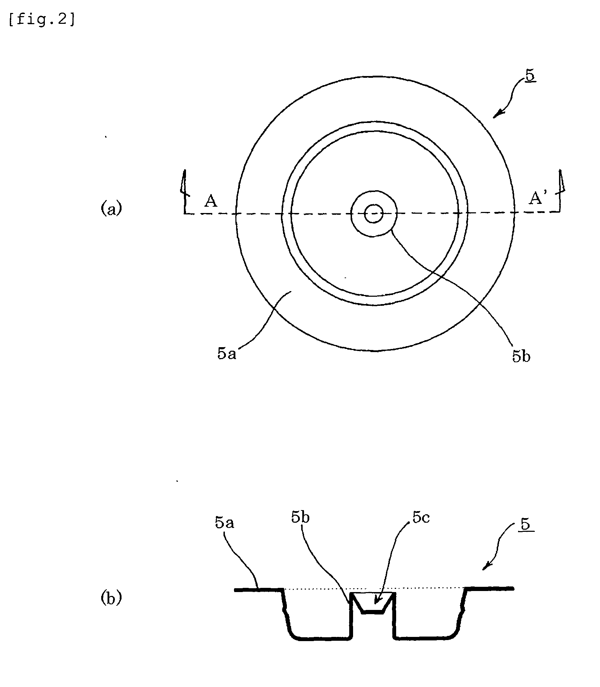

Fig. 2 is a diagram showing an example of the dissolution liquid-storing blister container 5 used in the present invention, where (a) is a top view and (b) is an A-A' sectional view of (a) As shown in the drawing, the blister container 5 has a flange portion 5a at the top of the outer circumference and a projected portion 5b at the center in the container.

At the time of using the transdermal drug device of the present invention, the device is applied to the skin with the adhesive 7 provided on the top surface of the expanded sheet 3, and the lid member 6 is broken by pushing the bottom of the blister container 5 by the projected portion 5b to migrate the dissolution liquid 4 to the drug-loaded member 2 through the dissolution liquid passing portion 8 to mix with the drug. And, a current is passed to the electrode 1b to ionize the drug solution to introduce it into the body through the skin. If the lid member 6 could not be broken sufficiently at this time, the dissolution liquid 4 is not migrated satisfactorily to the drug-loaded member 2 but partially remained in the blister container, and a desired drug efficacy cannot be expected.

As a result of devoted studies to increase a dissolution liquid migration rate and to decrease an amount of liquid remained in the blister container, the present inventors have found that it is quite effective to form the tip of the projected portion 5b into a recessed shape, and have completed the present invention.

Specifically, the blister container of the present invention has a recessed portion 5c formed at the tip of a projected portion 5b as exemplified in Fig. 2. Thus, the tip of the projected portion 5b is formed to have a recessed shape, so that when the bottom of the blister container 5 is pushed, initial contact between the projected portion 5b and the lid member 6 can be made to be not in a state of plane contact but a state of line contact, the lid member 6 can be subject to initial rupture effectively by the concentration of stress, and the lid member can be broken along a large area corresponding to the contour of the tip (or the contour of the recessed shape) of the projected portion 5b.

The lid member 6, which is sealed to the flange portion 5a of the blister container 5, tends to have a state that it is stretched taut when closer to the flange portion 5a but has a slackened state at the center portion, so that it is broken easier at a portion closer to the outer circumferential portion than to the center portion. Therefore, the lid member can be broken very easily and effectively by forming the tip of the projected portion 5b into the recessed shape as in the present invention and by causing the initial rupture at a portion deviated from the center portion of the lid member 6.

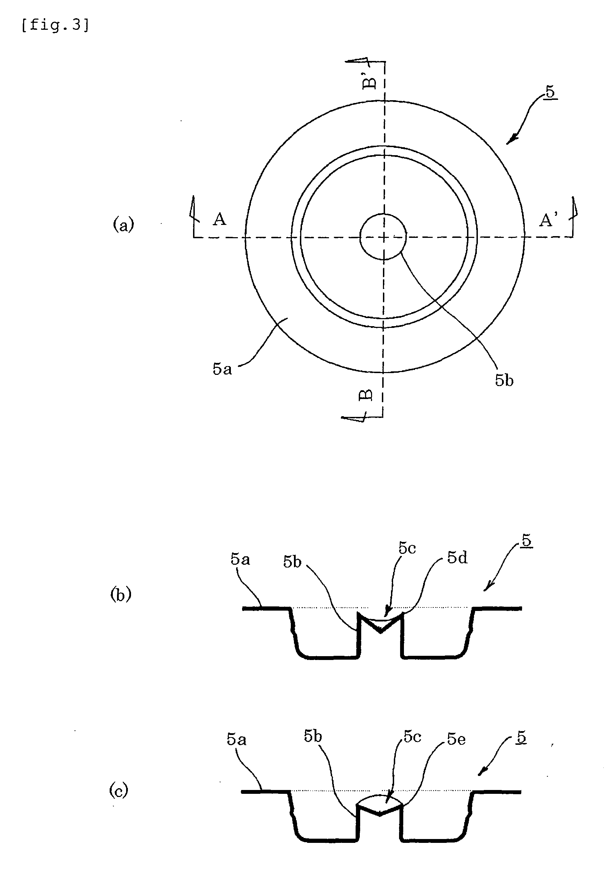

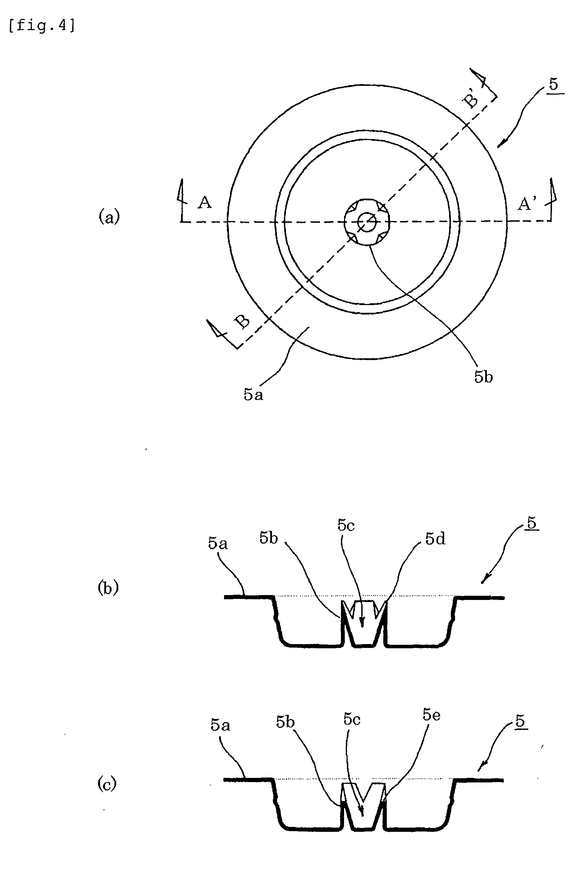

Other examples of the dissolution liquid-storing blister container 5 used in the present invention are shown in Fig. 3 through Fig. 5, where (a) is a top view, (b) is an A-A' sectional view of (a), and (c) is a B-B' sectional view of (a).

The example of Fig. 2 has the tip of the projected portion 5b formed to have the same height along the entire circumference, but as exemplified in Fig. 3 through Fig. 5, the outer circumference (or the outer circumference of the recessed shape) of the projected portion 5b may also be formed at a top 5d and a bottom 5e. By adopting such a form, the initial contact between the projected portion 5b and the lid member can be further changed from the line contact state to a point contact state, and the stress can be further concentrated to break through the lid member more effectively. As exemplified in Fig. 3 through Fig. 5, the top 5d and the bottom 5e are provided in plural to arrange them symmetrically on the outer circumference of the projected portion 5b, so that the lid member can be subject to initial rupture equally at the plural portions, and the lid member can be broken through along a large range more securely.

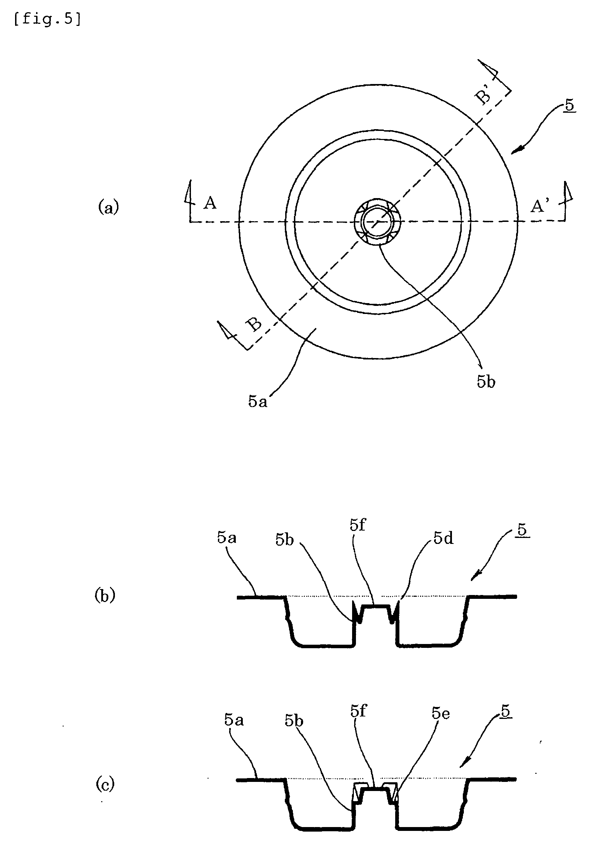

In the example shown in Fig. 5, a projected portion 5f lower than the top 5d is formed in the recessed portion 5c. By adopting such a style, even if the projected portion 5b is not pushed deep enough, the inside of the lid member which is undergone the initial rupture by the top 5d can be pushed by the projected portion 5f, so that the insufficient broken state can be avoided effectively.

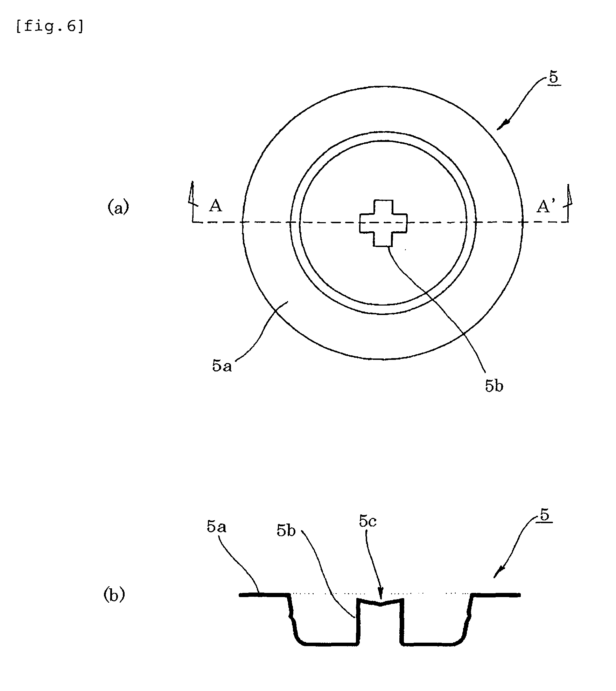

The contour of the projected portion 5b is not limited to the circular shape (cylindrical shape) exemplified in Fig. 2 through Fig. 5 but may have, for example, a polygonal columnar shape, a cross shape as exemplified in Fig. 6, or the like.

For the dissolution liquid-storing blister container 5, it is desired to use a material having a high steam barrier property to prevent the dissolution liquid from evaporating and decreasing its amount when the dissolution liquid 4 is charged and stored in the dissolution liquid-storing blister container 5. Specifically, examples of such a material include polyvinyl chloride, polyvinylidene chloride, polyethylene, polypropylene, polystyrene, polyamide, polymethylmethacrylate, polycarbonate, polyester, polyvinyl alcohol, polyvinyl acetate, thermoplastic elastomer, cyclic olefin polymer, cyclic olefin copolymer, a copolymer of ethylene with an organic carboxylic acid derivative having an ethylenically unsaturated bond, such as ethylene-methacrylate copolymer, ethylene-vinyl acetate copolymer, ethylene-acrylic acid copolymer and ethylene-vinyl acetate-methyl methacrylate copolymer, trifluoroethylene chloride resin, and the like. The dissolution liquid-storing blister container can be obtained by vacuum molding, compressed air molding or vacuum compressed air molding of a monolayer or a sheet formed of laminated multilayers.

### (Lid member)

The lid member 6 is preferably formed of a material excelling in a steam barrier property and a break-through characteristic such that permeation of moisture to the drug-loaded member 2 is prevented during storage and it is broken easily to mix the electrolyte 4 of the blister container 5 and the drug of the drug-loaded member 2 when used. Specifically, examples of such a material include polyester, polystyrene, polyolefin such as polypropylene, an ethylene-propylene copolymer, polyethylene and the like, and it is a uniaxially-stretched sheet or a sheet with an inorganic filler of calcium carbonate or the like blended into the above material, an aluminum foil or the like. And, a half-cut line may be formed in the lid member to facilitate its rupture.

As described above, the present invention is suitably applied to the iontophoresis device but not limited to it, and it is preferably applied to a drug device that a drug and a dissolution liquid are separately stored considering the storage stability of the drug, mixed at the time of use and absorbed through the skin or the mucosa. Such a device has a structure that the electrode holder 1 of Fig. 1 is a holder which is not provided with the electrode layer 1b and comprised of the base 1a only, and similar to the structure of the above-described iontophoresis device excepting such a structure.

### EXAMPLES

Specific examples of the present invention are described below but the present invention is not limited to them.

### [Example 1]

The iontophoresis device shown in Fig. 1 was produced as follows.

### (Formation of lid member-laminated electrode holder)

Conductive carbon and a conductive silver chloride paste ink were screen printed on a base 1a of a biaxially stretched polyethylene terephthalate (PET) film having a thickness of 75 µm to form an electrode layer 1b having a thickness of 30 µm.

A sealing layer having a thickness of 10 µm was formed by screen printing of an ink composed of a thermoplastic saturated copolymer polyester resin on a prescribed region such as a peripheral portion of the electrode layer 1b.

An adhesive layer was formed on the non-printed surface of the base by partial coating of an acrylic-based adhesive (trade name "SK-Dyne" produced by Soken Chemical & Engineering Co. , Ltd. ) on the portion bonded to the blister, and a hole which became a dissolution liquid passing portion 8 was formed by punching fabrication to obtain an electrode holder.

The lid member 6, which had a sealant resin (maleinated polypropylene) coated on a hard aluminum foil having a thickness of 20 µm, was laminated on the adhesive layer-formed side of the electrode holder to obtain a lid member-laminated electrode holder.

### (Formation of dissolution liquid-storing blister container)

The dissolution liquid-storing blister containers 5 shown in Fig. 3 and Fig. 6 were formed by vacuum molding of a resin sheet (trade name "SUMILITE" produced by Sumitomo Bakelite Co. Ltd.) for PTP (Press Through Package). These blister containers each have a height of 7 mm from the bottom to the flange 5a, a height of 6 mm to the top of the projected portion 5b, and a maximum size of 5 mm of the contour of the projected portion 5b.

### (Assembling of device)

An expanded olefin sheet 3 (trade name "Volara" produced by SEKISUI CHEMICAL CO., LTD.) and a drug-loaded nonwoven fabric 2 were positioned on the sealing layer which was formed on the electrode side of the lid member-laminated electrode holder and bonded mutually by heat sealing.

Subsequently, 650 mg of a dissolution liquid 4 was charged into the blister container 5, and the flange surface of the blister container was bonded to the lid member surface of the lid member-laminated electrode holder by impulse sealing to complete the iontophoresis device.

### (Measurement of solution migration rate)

The blister container 5 of the above iontophoresis device was turned upside, the circumference of the projected portion 5b was pushed to a depth of 4 mm (a depth that can be pushed by a finger easily, and the tip of the projected portion 5b is protruded 3 mm from the flange surface of the blister container) by means of a cylindrical indenter having a diameter of 7 mm, the solution remained in the container was measured in one minute after the indenter was pulled up, and the solution migration rate toward the drug-loaded member was calculated. The results are shown in Table 1.

### [Example 2]

A transdermal drug device was produced in the same manner as in Example 1 except that the electrode layer 1b was not formed, and the solution migration rate was measured. The results obtained were similar to those in Example 1.

**[Table 1]**

| Blister container | Remained solution amount (mg) | Absorbed amount (mg) | Migration rate (%) |
|---|---|---|---|
| Fig. 3 | 71 | 579 | 89.1 |
| Fig. 6 | 64 | 586 | 90.2 |

### [Comparative Examples]

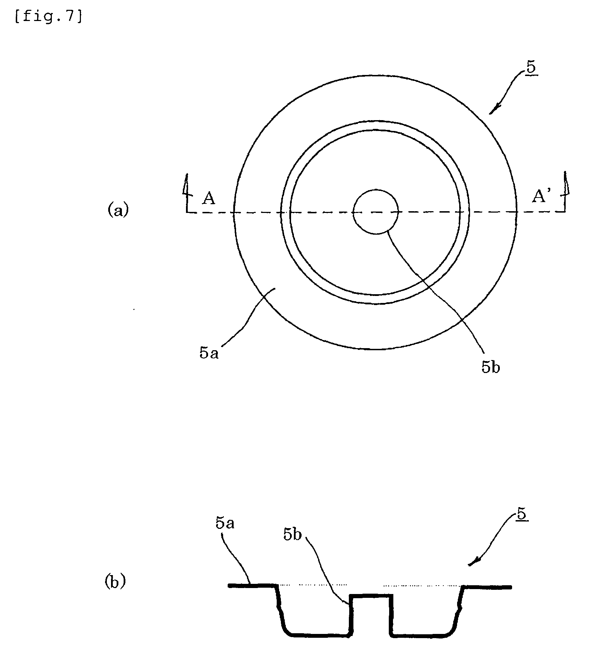

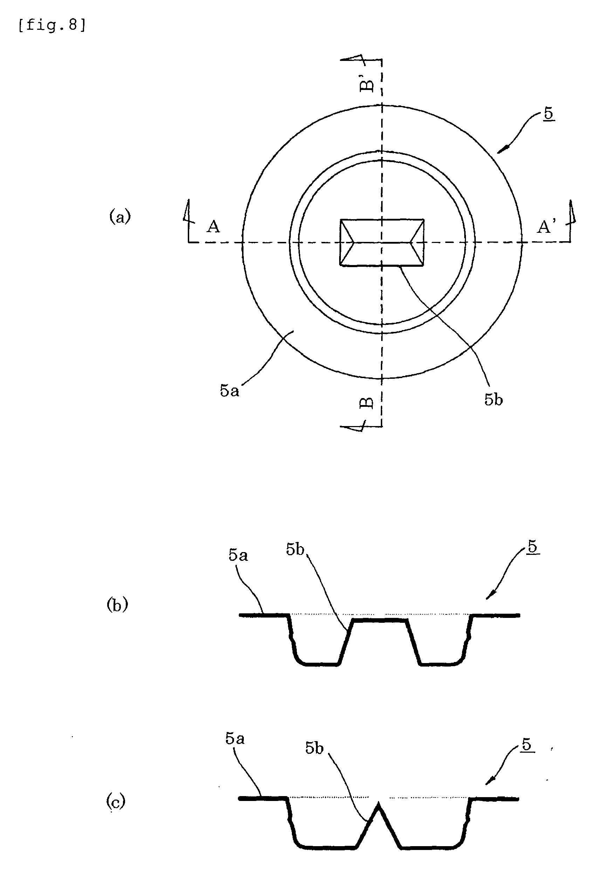

The iontophoresis devices were produced in the same manner as in Example 1 except that the dissolution liquid-storing blister containers shown in Fig. 7 and Fig. 8 were used, and the solution migration rates were measured. The results are shown in Table 2. The blister containers had a height of 7 mm from the bottom to the flange 5a, a height of 6 mm up to the top of the projected portion 5b, and a maximum size of 5 mm of the contour of the projected portion 5b similar to those of Example 1.

**[Table 2]**

| Blister container | Remained solution amount (mg) | Absorbed amount (mg) | Migration rate (%) |
|---|---|---|---|
| Fig. 7 | 323 | 327 | 50.3 |
| Fig. 8 | 144 | 506 | 77.8 |

It is apparent from the results of Examples 1, 2 and the comparative examples that the transdermal drug device of the present invention using the dissolution liquid-storing blister container, which has the tip of the projected portion formed to have a recessed shape and the top and bottom formed on the outer circumference of the projected portion, has a very high dissolution liquid migration rate of about 90%.

Meanwhile, when the dissolution liquid-storing blister container having the cylindrical projected portion shown in Fig. 7 is used, the dissolution liquid migration rate is less than 80%. It was because the projected portion and the lid member were in plane contact to each other, and the lid member could not be broken satisfactorily.

When the dissolution liquid-storing blister container shown in Fig. 8 is used, the dissolution liquid migration rate is less than 80%. It is because the initial contact between the projected portion and the lid member can be made to be a line contact and the lid member can be subject to initial rupture effectively but has a very small breaking area.

In the above examples, the aluminum lid member was used as the lid member, but the same results are obtained by using a resin lid.

### BRIEF DESCRIPTION OF THE DRAWINGS

[Fig. 1] Diagram showing an embodiment of a transdermal drug device according to the present invention, where (a) is a sectional view and (b) is a perspective view.
[Fig. 2] Diagram showing an example of a dissolution liquid-storing blister container used for the transdermal drug device of the present invention, where (a) is a top view and (b) is an A-A' sectional view of (a).
[Fig. 3] Diagram showing another example of the dissolution liquid-storing blister container used for the transdermal drug device of the present invention, where (a) is a top view, (b) is an A-A' sectional view of (a) and (c) is a B-B' sectional view of (a).
[Fig. 4] Diagram showing another example of the dissolution liquid-storing blister container used for the transdermal drug device of the present invention, where (a) is a top view, (b) is an A-A' sectional view of (a) and (c) is a B-B' sectional view of (a).
[Fig. 5] Diagram showing another example of the dissolution liquid-storing blister container used for the transdermal drug device of the present invention, where (a) is a top view, (b) is an A-A' sectional view of (a) and (c) is a B-B' sectional view of (a).
[Fig. 6] Diagram showing another example of the dissolution liquid-storing blister container used for the transdermal drug device of the present invention, where (a) is a top view and (b) is an A-A' sectional view of (a).
[Fig. 7] Diagram showing the dissolution liquid-storing blister container used in Comparative Example, where (a) is a top view and (b) is an A-A' sectional view of (a).
[Fig. 8] Diagram showing the dissolution liquid-storing blister container used in Comparative Example, where (a) is a top view, (b) is an A-A' sectional view of (a) and (c) is a B-B' sectional view of (a).

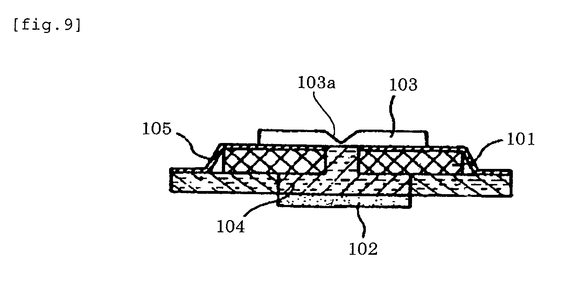

[Fig. 9] Explanatory view of a conventional example.

### EXPLANATION OF REFERENCE NUMERALS

1: Electrode holder
1a: Base of electrode holder
1b: Electrode layer
2: Drug-loaded member
3: Expanded sheet
4: Dissolution liquid
5: Dissolution liquid-storing container (Dissolution liquid-storing blister container)
5a: Flange
5b: Projected portion
5c: Recessed portion
5d: Top
5e: Bottom
5f: Projected portion lower than top 5d
6: Lid member
7: Adhesive
8: Dissolution liquid passing portion
101: Electrode layer
102: Drug-containing layer
103: Electrolyte capsule
103a: Projection
104: Water-containing layer
105: Sealing cover

## Claims

1. A transdermal drug device, comprising a holder having a dissolution liquid passing hole, a drug-loaded member provided on one side surface of the holder, and a dissolution liquid-storing container provided on the other side surface of the holder via a lid member for covering the dissolution liquid passing hole, wherein the dissolution liquid-storing container has a projected portion which is opposed to the dissolution liquid passing hole, and the tip of the projected portion is formed on a recessed surface.

2. The transdermal drug device according to claim 1, wherein the projected portion has a top and bottom on its outer circumference.

3. The transdermal drug device according to claim 2, wherein the top and the bottom are respectively provided in plural and arranged on the outer circumference of the projected portion in contrast to one another.

4. The transdermal drug device according to any of claims 1 through 3, wherein the holder is an iontophoresis device which is provided with an electrode on the drug-loaded member side.
